# EUROPÄISCHE PATENTSCHRIFT

(11) **EP 2 522 284 B1**
(45) Veröffentlichungstag und Bekanntmachung des Hinweises auf die Patenterteilung: **28.08.2019**
(21) Anmeldenummer: 12003420.2
(22) Anmeldetag: 04.05.2012
(51) Int. Cl.: A61B 17/29

(54) **Medizinisches Rohrschaftinstrument**
Medical tube shaft instrument
Instrument médical à tige cylindrique

(30) Priorität: 13.05.2011 DE 102011075785
(43) Veröffentlichungstag der Anmeldung: 14.11.2012
(73) Patentinhaber: Karl Storz SE & Co. KG, 78532 Tuttlingen (DE)
(72) Erfinder: Bacher, Uwe, 78532 Tuttlingen (DE); Wittner, Stefan, 78532 Tuttlingen (DE)

(56) Entgegenhaltungen:
- DE-C1- 19 853 305
- DE-C1- 19 855 968
- US-A- 5 395 375
- US-A- 5 569 298
- US-A1- 2002 165 538

## Beschreibung

Die Erfindung betrifft ein medizinisches Rohrschaftinstrument. Ein medizinisches Rohrschaftinstrument und ein Herstellungsverfahren sind aus der DE 198 55 968 C1 bekannt.

Derartige Rohrschaftinstrumente werden in der minimal-invasiven Chirurgie als Operationsinstrumente eingesetzt, um operative Eingriffe durch eine kleine Körperinzision unter endoskopischer Kontrolle durchzuführen. Für unterschiedliche durchzuführende operative Eingriffe im menschlichen oder tierischen Körper werden solche Rohrschaftinstrumente mit unterschiedlichen Funktionen ausgestattet.

Unter einem Rohrschaftinstrument im Sinne der vorliegenden Erfindung wird z. B. eine Zange zum Schneiden und/oder Fassen und/oder ein Nadelhalter verstanden. Die Maulteilanordnung kann ein bewegliches Maulteil haben, das mit einer Schneide eines zweiten, am Rohr befestigten Maulteils schneidend zusammenwirkt. Die Maulteilanordnung kann auch stumpfe Maulteile zum Ergreifen von Gewebe haben. Im Falle eines Nadelhalters sind die Maulteile dafür ausgebildet, eine Nadel lösbar zu halten, um im Operationsgebiet eine Naht zum Verbinden von Gewebe herzustellen.

Zur Betätigung der Maulteilanordnung befindet sich am proximalen Ende des Rohres ein Handstück, das über ein langgestrecktes Kraftübertragungselement, normalerweise in Form einer Zugstange, kraftschlüssig mit dem beweglichen Maulteil verbunden ist. Eine manuelle Betätigung der Griffe des Handstücks bewirkt eine Bewegung der Maulteilanordnung am distalen Ende des Rohres, um die gewünschte Aufgabe wie z. B. Schneiden oder Halten von Gewebe oder Führen einer Nadel durch das Gewebe durchzuführen.

Für die minimal-invasive Chirurgie, insbesondere bei der Operation an Kleinkindern oder auch bei Eingriffen in bestimmten Operationsbereichen, wie im Kopfbereich, ist es mitunter erforderlich, solche Rohrschaftinstrumente mit einem sehr geringen Rohrdurchmesser auszubilden.

Außerdem besteht bei Rohrschaftinstrumenten die Anforderung, dass sie der Länge nach mit Spülflüssigkeit durchspülbar sein sollen, um sie zum Reinigen nicht vollständig zerlegen zu müssen. Um den zum Spülen erforderlichen Freiraum zu schaffen, wird in dem o. g. Stand der Technik vorgeschlagen, die Zugstange allgemein um einiges dünner zu machen als der Innendurchmesser des Rohres, aber in mindestens einem Abschnitt entlang ihrer Länge drei radial verteilt angeordnete Abstützvorsprünge vorzusehen, die einerseits das Kraftübertragungselement zentrisch im Rohr abstützen, damit es sich nicht leicht verbiegen kann, andererseits aber auch in diesen Abschnitten Raum zum Durchspülen des Rohres freilassen, um Sterilitäts- und Hygieneanforderungen zu genügen.

Dokument US 5 569 298 A offenbart ein medizinisches Rohrschaftinstrument mit einem geraden langgestreckten Rohr und mit einem stangenförmigen Kraftübertragungselement, wobei das Rohr an einem distalen Ende eine Maulteilanordnung trägt, wobei das Kraftübertragungselement genau einen Abschnitt aufweist, in dem es in dem Rohr zentriert wird, dieser Abschnitt in der Nähe des distalen Endes des Kraftübertragungselements angeordnet ist und dieser Abschnitt durch Verformen des Kraftübertragungselements senkrecht zu seiner Längsachse ausgebildet ist.

Der vorliegenden Erfindung liegt die Aufgabe zu Grunde, das bekannte Rohrschaftinstrument dahingehend zu verbessern, dass es einerseits besonders einfach herstellbar und ausreichend stabil ist und andererseits besonders strenge Sterilitäts- und Hygieneanforderungen erfüllen kann.

Diese Aufgabe wird durch ein medizinisches Rohrschaftinstrument gemäß Anspruch 1 gelöst.

Die Erfindung beruht zum einen auf der Erkenntnis, dass man mit nur einem Abschnitt auskommt, in dem das Kraftübertragungselement in dem Rohr zentriert wird, wenn man diesen Abschnitt in der Nähe des distalen Endes des Kraftübertragungselements anordnet. Es hat sich gezeigt, dass in der Regel nur am distalen Ende Querkräfte auftreten, die in der Lage wären, das Kraftübertragungselement zu verbiegen, so dass eine Abstützung in diesem Bereich genügt.

Zum anderen ermöglicht es die Erfindung, den Abschnitt, in dem das Kraftübertragungselement in dem Rohr zentriert wird, auf besonders einfache Weise herzustellen, nämlich durch partielles Verformen des Kraftübertragungselements senkrecht zu seiner Längsachse, d. h. durch einen einfachen Pressvorgang.

Im Gegensatz dazu ist in dem o. g. Stand der Technik vorgesehen, Abstützvorsprünge am Kraftübertragungselement entweder aufwändig materialabtragend oder durch Rollen oder Ziehen eines Rundmaterials herzustellen. Im letzteren Fall ändert sich die Querschnittsfläche, weil das Material dabei gestreckt wird. Dadurch ändert sich auch der für das Spülen zur Verfügung stehende Strömungsquerschnitt über die Länge des Kraftübertragungselements, was für die Effizienz der Spülung ungünstig ist, weil die Strömungsgeschwindigkeit über die Länge variiert.

Hingegen kann bei dem erfindungsgemäßen Kraftübertragungselement leicht dafür gesorgt werden, dass die Übergänge zwischen dem Abschnitt, in dem das Kraftübertragungselement in dem Rohr zentriert wird, und dem übrigen Kraftübertragungselement sanft, das heißt nicht sprunghaft, sind und dass die quer zu seiner Längsachse gemessene Querschnittsfläche des Kraftübertragungselements über seine gesamte Länge ungefähr konstant ist. Somit ist auch der Strömungsquerschnitt überall ungefähr konstant, so dass mit einem gegebenen Spüldruck eine maximale Durchflussmenge mit maximaler Spüleffizienz erzielt werden kann. Und da der Abschnitt, in dem das Kraftübertragungselement in dem Rohr zentriert wird, nicht durch irgendwelche Querschnittsänderungen oder abrupte Übergänge mechanisch geschwächt wird, kann das Kraftübertragungselement über seine gesamte Länge mit der minimalen, für die benötigte Festigkeit noch ausreichenden Querschnittsfläche hergestellt werden, so dass überall ein maximaler Spülquerschnitt erzielt werden kann.

Es gibt zwei zueinander entgegengesetzt nach außen weisende Abstützvorsprünge, die durch die in Längsrichtung verlaufenden Ränder einer ebenen Platte gebildet werden, zu der das Kraftübertragungselement in dem Abschnitt flachgepresst wird.

Wenn die Maulteilanordnung zwei Maulteile umfasst, von denen mindestens eines um eine Schwenkachse schwenkbar am distalen Ende des Rohres gelagert ist, wird das plattenförmige Kraftübertragungselement vorteilhaft so angeordnet, dass seine Ebene senkrecht zu der Schwenkachse verläuft. In diesem Fall wirken irgendwelche Querkräfte auf das Kraftübertragungselement im Wesentlichen nur senkrecht zu der Schwenkachse und nur in diesem Bereich, und diese Querkräfte werden durch die entsprechend angeordnete Platte gut auf das Rohr übertragen, ohne dass sich das Kraftübertragungselement verbiegen kann.

In einer praktischen Ausführungsform hat die ebene Platte ungefähr die Form eines flachen Quaders, dessen Dicke ungefähr ein Drittel bis die Hälfte des Durchmessers des Kraftübertragungselements beträgt und dessen Länge in Längsrichtung des Kraftübertragungselements ungefähr oder mindestens das Doppelte des allgemeinen Durchmessers des Kraftübertragungselements beträgt.

Die Ränder der Platte oder anders gebildete Abstützvorsprünge können abgerundet oder angefast sein, derart, dass sie eine Konkavität mit geringerer Krümmung als die Krümmung der Innenwand des Rohres haben. Dadurch wird erreicht, dass sich die Abstützvorsprünge nicht flächig, sondern linienförmig an der Innenwand des Rohres abstützen, so dass es keine für Spülflüssigkeit unzugänglichen Toträume gibt.

In einer bevorzugten Ausfuhrungsform ist das Rohrschaftinstrument eine mikrochirurgische Zange, wobei das Kraftübertragungselement eine Zugstange ist.

Es folgt eine Beschreibung eines Ausführungsbeispiels anhand der Figuren. Es zeigen:
- Figuren 1a und 1b: zueinander senkrechte ebene Längsschnittansichten durch einen Zangenaufsatz einer mikrochirurgischen Rohrschaftzange, d. h. ohne deren Handstück; und
- Figuren 2a und 2b: zueinander senkrechte vergrößerte Längsansichten der Zugstange des Zangenaufsatzes von Figuren 1a und 1b.

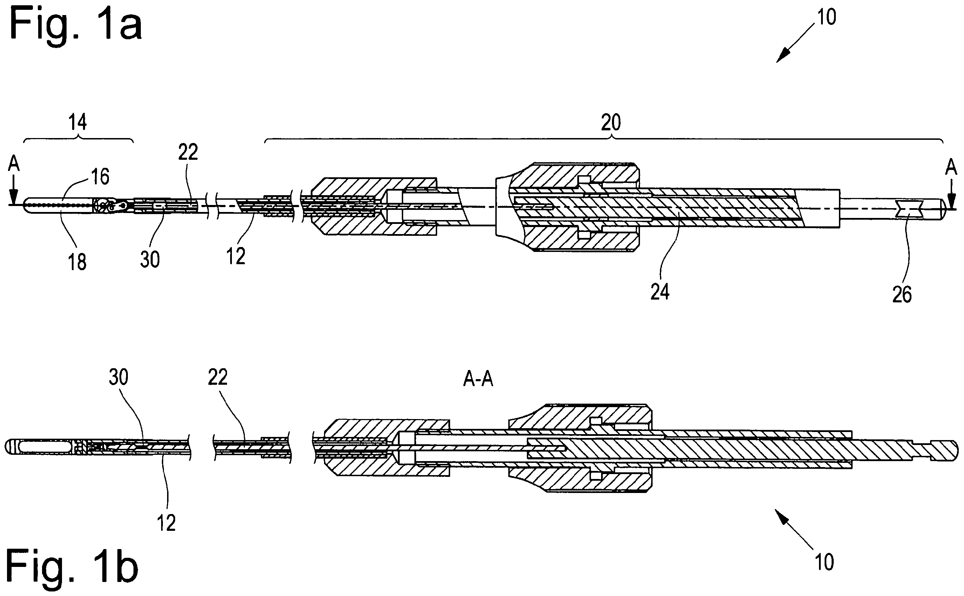

In Figuren 1a und 1b ist ein mit dem allgemeinen Bezugszeichen 10 versehener Zangenaufsatz einer mikrochirurgischen Rohrschaftzange dargestellt.

Der Zangenaufsatz 10 enthält ein gerades langgestrecktes starres Rohr 12, das am distalen Ende eine Maulteilanordnung 14 trägt, die zwei relativ zueinander schwenkbare Maulteile 16 und 18 aufweist. Beide Maulteile 16 und 18 können schwenkbar sein, oder es ist nur eines schwenkbar, während das andere unbeweglich am Rohr 12 befestigt ist. Die Maulteilanordnung 14 ist hier als eine Greifzange dargestellt, kann mit entsprechend ausgebildeten Maulteilen aber auch eine Schneidzange sein.

Das Rohr 12 sitzt an seinem proximalen Ende in einem Ende eines langgestreckten, mehr oder weniger rohrförmigen Halters 20, der an einem nicht gezeigten Handstück anbringbar ist, das Griffe hat, von denen mindestens einer beweglich ist.

Mittig durch das Rohr 12 hindurch verläuft eine Zugstange 22, welche an einem distalen Ende über winzige Hebel mit einem Maulteil oder mit beiden Maulteilen 16, 18 gekoppelt ist. An ihrem proximalen Ende erstreckt sich die Zugstange 22 ein Stück weit axial in den Halter 20 hinein und ist dort kraftschlüssig mit einer weiteren, dickeren Stange 24 gekoppelt, die sich axial durch den Halter 20 hindurch erstreckt und an dessen proximalem Ende austritt.

Die weitere Stange 24 hat an ihrem proximalen Ende einen Kopplungsteil 26 zur Kopplung mit den Griffen des Handstücks. Die Zugstange 22 und die weitere Stange 24 bilden eine Wirkverbindung zwischen der Maulteilanordnung 14 und den Griffen des Handstücks, an dem der Halter 20 angebracht wird, um die Rohrschaftzange mit einer Hand zu halten und dabei die Maulteile 16 und 18 manuell öffnen bzw. schließen zu können, eventuell gegen die Kraft einer Feder.

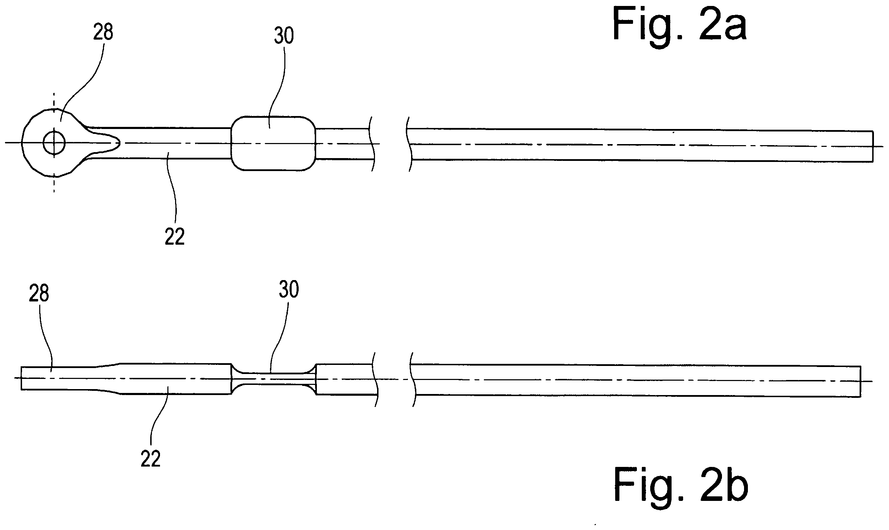

Die in Figuren 2a und 2b vergrößert gezeichnete Zugstange 22 hat allgemein einen Durchmesser, der um so viel kleiner als der Innendurchmesser des Rohres 12 ist, dass ein ausreichender Freiraum zum axialen Durchspülen des Rohres 12 bei eingebauter Zugstange 22 bleibt.

Zum Durchspülen wird der Zangenaufsatz 10 in eine Waschmaschine für medizinische Instrumente gelegt und an einem Ende über einen Schlauch und Adapter mit einem Fluidanschluss verbunden, der unter geringen Druck stehende Spülflüssigkeit bereitstellt.

Wie man in Fig. 2 erkennt, hat die Zugstange 22 an ihrem distalen Ende einen gelochten Kopf 28 für eine Gelenkverbindung mit den Hebeln der Maulteile 16 bzw. 18.

Nicht weit von ihrem distalen Ende entfernt ist die Zugstange 22 in einem Abschnitt, dessen axiale Länge ungefähr das Dreifache der allgemeinen Dicke der Zugstange 22 beträgt, zu einer ebenen Platte 30 flachgepresst. Das beim Pressen verdrängte Material ist im Wesentlichen senkrecht zur Längsrichtung der Zugstange 22 ausgewichen, und zwar so weit, dass die Breite der Platte 30 dem Innendurchmesser des Rohres 12 entspricht, so dass die Zugstange 22 durch die Platte 30 zentrisch im Rohr 12 geführt wird. Das heißt, die in Längsrichtung des Rohres 12 verlaufenden Ränder der Platte 30 bilden entsprechende Abstützelemente.

Mittels geeigneter Stempel könnte auf ähnliche Weise eine andere Abstützstruktur erzeugt werden, z. B. mit in einem Dreieck oder sternförmig angeordneten Abstützelementen.

In dem gezeigten Ausführungsbeispiel ist die Zugstange 22 allgemein 4 mm dick und ist die Platte 30 ungefähr 11 mm lang, 1,15 mm dick und 7 mm breit, wobei ihre Breite an ein Rohr mit 7 mm Innendurchmesser angepasst ist, also geringfügig kleiner sein kann. Aus diesen Maßen ergibt sich, dass die Platte 30 beinahe dieselbe Querschnittsfläche senkrecht zur Längsrichtung hat wie die Zugstange 22. Ein kleiner Unterschied ist unschädlich und kann einer materialabtragenden Nacharbeitung der Ränder der Platte 30 und/oder einer geringfügigen Materialstreckung in Längsrichtung beim Pressen geschuldet sein.

Wie man in Figuren 1a und 1b erkennt, steht die Ebene der Platte 30 senkrecht zu der Schwenkachse der Maulteile 16, 18 der Maulteilanordnung 14. Somit stützt die Platte 30 die Zugstange 22 in der optimalen Richtung und genau in der Richtung, in der am ehesten Querkräfte auftreten, in der Innenwand des Rohres 12 ab.

Obwohl in den Zeichnungen nicht gezeigt, können die Ränder der Platte 30 senkrecht zur Längsrichtung der Zugstange 22 mit Rundungen oder Fasen versehen werden, um eine Konkavität mit geringerer Krümmung als die Krümmung der Innenwand des Rohres 12 herzustellen, damit sich die Platte 30 nur linienförmig und nicht mehr oder weniger flächig an der Innenwand des Rohres 12 abstützt.

### Bezugszeichen

- 10: Zangenaufsatz
- 12: Rohr
- 14: Maulteilanordnung
- 16, 18: Maulteil
- 20: Halter
- 22: Zugstange
- 24: weitere Stange
- 26: Kopplungsteil
- 28: gelochter Kopf der Zugstange 22
- 30: ebene Platte bzw. Abschnitt, in dem die Zugstange 22 flachgepresst ist

## Patentansprüche

1. Medizinisches Rohrschaftinstrument (10)
mit einem geraden langgestreckten Rohr (12) und
mit einem stangenförmigen Kraftübertragungselement (22), das sich der Länge nach durch das Rohr (12) erstreckt und entlang der Länge des Rohres (12) darin verschiebbar ist,
wobei das Rohr (12) an einem distalen Ende eine Maulteilanordnung (14) trägt, die außerdem mit dem Kraftübertragungselement (22) gekoppelt ist, um durch Verschieben des Kraftübertragungselementes (22) entlang des Rohres (12) betätigt zu werden,
wobei das Rohr (12) an einem proximalen Ende an einem Handstück mit zueinander beweglichen Griffen befestigt ist oder dafür eingerichtet ist, an einem derartigen Handstück angebracht zu werden, derart, dass das Kraftübertragungselement (22) entlang des Rohres (12) verschoben wird, wenn das Handstück manuell betätigt wird, wobei das Kraftübertragungselement (22) über den größten Teil seiner Länge einen kleineren Außendurchmesser als der Innendurchmesser des Rohres (12) hat, um einen ringförmigen Spülkanal zwischen dem Kraftübertragungselement (22) und dem Rohr (12) auszubilden, und
wobei das Kraftübertragungselement (22) in mindestens einem Abschnitt entlang seiner Länge derart ausgebildet ist, dass es in diesem Abschnitt in dem Rohr (12) zentriert wird und außerdem ein Freiraum zum Durchspülen des Rohres (12) bei eingebautem Kraftübertragungselement (22) bleibt,
wobei,
das Kraftübertragungselement (22) genau einen Abschnitt (30) aufweist, in dem es in dem Rohr (12) zentriert wird, wobei dieser Abschnitt (30) in der Nähe des distalen Endes des Kraftübertragungselements (22) angeordnet ist und dieser Abschnitt (30) durch Verformen des Kraftübertragungselements (22) senkrecht zu seiner Längsachse ausgebildet ist, wobei der Abschnitt (30), in dem das Kraftübertragungselement (22) in dem Rohr (12) zentriert wird, zwei radial nach außen weisende Abstützvorsprünge enthält, und wobei der Abschnitt (30), in dem das Kraftübertragungselement (22) in dem Rohr (12) zentriert wird, genau zwei zueinander entgegengesetzt nach außen weisende Abstützvorsprünge enthält, die durch die in Längsrichtung verlaufenden Ränder einer ebenen Platte (30) gebildet werden, zu der das Kraftübertragungselement (22) in dem Abschnitt (30) flachgepresst ist, **dadurch gekennzeichnet, dass** das stangenförmigen Kraftübertragungselement (22) einstückig ist.

2. Medizinisches Rohrschaftinstrument (10) nach Anspruch 1, **dadurch gekennzeichnet, dass** die Übergange zwischen dem Abschnitt (30), in dem das Kraftübertragungselement (22) in dem Rohr (12) zentriert wird, und dem übrigen Kraftübertragungselement (22) sanft sind und dass die quer zur Längsachse des Kraftübertragungselements (22) gemessene Querschnittsfläche des Kraftübertragungselements über seine gesamte Länge ungefähr konstant ist.

3. Medizinisches Rohrschaftinstrument (10) nach einem der vorhergehenden Ansprüche, **dadurch gekennzeichnet, dass** die Maulteilanordnung (14) zwei Maulteile (16, 18) umfasst, von denen mindestens eines um eine Achse, die senkrecht zu der Ebene der Platte (30) des Kraftübertragungselements (22) verläuft, schwenkbar am distalen Ende des Rohres (12) gelagert ist.

4. Medizinisches Rohrschaftinstrument (10) nach einem der vorhergehenden Ansprüche, **dadurch gekennzeichnet, dass** die Platte (30) ungefähr die Form eines flachen Quaders hat, dessen Dicke ungefähr ein Drittel bis die Hälfte des Durchmessers des Kraftübertragungselements (22) beträgt und dessen Länge in Längsrichtung des Kraftübertragungselements (22) ungefähr oder mindestens das Doppelte des allgemeinen Durchmessers des Kraftübertragungselements (22) beträgt.

5. Medizinisches Rohrschaftinstrument (10) nach einem der vorhergehenden Ansprüche, **dadurch gekennzeichnet, dass** die Abstützvorsprünge so geformt sind, dass ihre Kontaktbereiche mit der Innenwand des Rohres (12) linienförmig sind.

6. Medizinisches Rohrschaftinstrument (10) nach einem der vorhergehenden Ansprüche, **dadurch gekennzeichnet, dass** es eine mikrochirurgische Zange ist, wobei das Kraftübertragungselement (22) eine Zugstange ist.

## Claims

1. Medical tubular shaft instrument (10),
with a straight elongate tube (12) and
with a rod-shaped force transmission element (22) which extends lengthwise through the tube (12) and which is slidable therein along the length of the tube (12),
wherein the tube (12) bears, at a distal end, a jaw member arrangement (14), which is moreover coupled to the force transmission element (22) in order to be actuated by sliding of the force transmission element (22) along the tube (12),
wherein the tube (12) is secured, at a proximal end, to a handpiece with grips movable towards each other or is configured to be attached to such a handpiece in such a way that the force transmission element (22) is slid along the tube (12) when the handpiece is manually actuated,
wherein the force transmission element (22), over most of its length, has an external diameter smaller than the internal diameter of the tube (12), in order to form an annular flushing channel between the force transmission element (22) and the tube (12), and
wherein the force transmission element (22), in at least one portion along its length, is configured in such a way that it is centred in the tube (12) in this portion and in addition a free space remains for flushing through the tube (12) when the force transmission element (22) is installed therein,
wherein the force transmission element (22) has precisely one portion (30) in which it is centred in the tube (12), wherein this portion (30) is arranged near the distal end of the force transmission element (22), and this portion (30) is formed by deformation of the force transmission element (22) perpendicularly to its longitudinal axis, wherein the portion (30), in which the force transmission element (22) is centred in the tube (12), has two radially outwardly facing support projections, and wherein the portion (30), in which the force transmission element (22) is centred in the tube (12), has precisely two outwardly facing support projections arranged opposite each other and formed by the longitudinally extending edges of a plane plate (30) into which the force transmission element (22) is pressed flat in the portion (30),
**characterized in that** the rod-shaped force transmission element (22) is in one piece.

2. Medical tubular shaft instrument (10) according to Claim 1, **characterized in that** the transitions between the portion (30), in which the force transmission element (22) is centred in the tube (12), and the rest of the force transmission element (22) are smooth, and **in that** the cross-sectional area of the force transmission element, measured transversely with respect to the longitudinal axis of the force transmission element (22), is approximately constant over its entire length.

3. Medical tubular shaft instrument (10) according to either of the preceding claims, **characterized in that** the jaw member arrangement (14) comprises two jaw members (16, 18), of which at least one is mounted at the distal end of the tube (12) in such a way as to be pivotable about an axis that runs perpendicular to the plane of the plate (30) of the force transmission element (22).

4. Medical tubular shaft instrument (10) according to one of the preceding claims, **characterized in that** the plate (30) has approximately the shape of a flat parallelepiped whose thickness is about one third to one half of the diameter of the force transmission element (22) and whose length in the longitudinal direction of the force transmission element (22) is approximately or at least twice the general diameter of the force transmission element (22).

5. Medical tubular shaft instrument (10) according to one of the preceding claims, **characterized in that** the support projections are shaped in such a way that their contact regions with the inner wall of the tube (12) are linear.

6. Medical tubular shaft instrument (10) according to one of the preceding claims, **characterized in that** the instrument is a pair of microsurgical forceps, wherein the force transmission element (22) is a pull rod.

## Revendications

1. Instrument médical à tige cylindrique (10) comprenant un tube droit allongé (12) et un élément de transfert de force (22) en forme de tige qui s'étend sur sa longueur à travers le tube (12) et qui peut être coulissé dans celui-ci le long de la longueur du tube (12),
le tube (12) portant, à une extrémité distale, un agencement de partie formant mâchoire (14) qui est en outre accouplé à l'élément de transfert de force (22) afin d'être actionné par déplacement de l'élément de transfert de force (22) le long du tube (12),
le tube (12) étant fixé au niveau d'une extrémité proximale à une pièce à main avec des poignées déplaçables l'une par rapport à l'autre ou étant prévu pour être monté sur une pièce à main de ce type de telle sorte que l'élément de transfert de force (22) soit coulissé le long du tube (12) lorsque la pièce à main est actionnée manuellement, l'élément de transfert de force (22) présentant sur la majeure partie de sa longueur un plus petit diamètre extérieur que le diamètre intérieur du tube (12) afin de créer un canal de rinçage de forme annulaire entre l'élément de transfert de force (22) et le tube (12), et
l'élément de transfert de force (22) étant réalisé dans au moins une portion le long de sa longueur de telle sorte qu'il soit centré dans le tube (12) dans cette portion et en outre qu'un espace libre subsiste pour le rinçage du tube (12) lorsque l'élément de transfert de force (22) est installé, l'élément de transfert de force (22) présentant exactement une portion (30) dans laquelle il est centré dans le tube (12), cette portion (30) étant disposée à proximité de l'extrémité distale de l'élément de transfert de force (22) et cette portion (30) étant réalisée par déformation de l'élément de transfert de force (22) perpendiculairement à son axe longitudinal, la portion (30) dans laquelle l'élément de transfert de force (22) est centré dans le tube (12) contenant deux saillies de support orientées radialement vers l'extérieur, et
la portion (30) dans laquelle l'élément de transfert de force (22) est centré dans le tube (12) contenant exactement deux saillies de support orientées vers l'extérieur de manière opposée l'une à l'autre, qui sont formées par les bords s'étendant dans la direction longitudinale d'une plaque plane (30) vers laquelle l'élément de transfert de force (22) est pressé à plat dans la portion (30),
**caractérisé en ce que**
l'élément de transfert de force en forme (22) de tige est réalisé d'une seule pièce.

2. Instrument médical à tige cylindrique (10) selon la revendication 1, **caractérisé en ce que** les transitions entre la portion (30), dans laquelle l'élément de transfert de force (22) est centré dans le tube (12), et le reste de l'élément de transfert de force (22) sont douces et **en ce que** la surface en section transversale de l'élément de transfert de force, mesurée transversalement à l'axe longitudinal de l'élément de transfert de force (22), est approximativement constante sur toute sa longueur.

3. Instrument médical à tige cylindrique (10) selon l'une quelconque des revendications précédentes, **caractérisé en ce que**
l'agencement de partie formant mâchoire (14) comprend deux parties formant mâchoire (16, 18) dont au moins une est supportée de manière à pouvoir pivoter au niveau de l'extrémité distale du tube (12) autour d'un axe qui s'étend perpendiculairement au plan de la plaque (30) de l'élément de transfert de force (22).

4. Instrument médical à tige cylindrique (10) selon l'une quelconque des revendications précédentes, **caractérisé en ce que**
la plaque (30) présente approximativement la forme d'un parallélépipède plat dont l'épaisseur vaut approximativement entre un tiers et la moitié du diamètre de l'élément de transfert de force (22) et dont la longueur dans la direction longitudinale de l'élément de transfert de force (22) vaut approximativement ou au moins le double du diamètre global de l'élément de transfert de force (22).

5. Instrument médical à tige cylindrique (10) selon l'une quelconque des revendications précédentes, **caractérisé en ce que**
les saillies de support sont formées de telle sorte que leurs zones de contact avec la paroi interne du tube (12) aient une forme linéaire.

6. Instrument médical à tige cylindrique (10) selon l'une quelconque des revendications précédentes, **caractérisé en ce qu'**il s'agit d'une pince de microchirurgie, l'élément de transfert de force (22) étant une pince de traction.
